# EUROPÄISCHE PATENTSCHRIFT

(11) **EP 1 824 445 B1**
(45) Veröffentlichungstag und Bekanntmachung des Hinweises auf die Patenterteilung: **02.07.2008**
(21) Anmeldenummer: 05818699.0
(22) Anmeldetag: 16.12.2005
(51) Int. Cl.: A61K 8/22, A61K 8/55, A61Q 11/00, A61K 8/67

(54) **ZAHNBLEICHMITTEL**
TEETH WHITENING AGENTS
AGENTS DE BLANCHIMENT DES DENTS

(30) Priorität: 17.12.2004 DE 102004061646
(43) Veröffentlichungstag der Anmeldung: 29.08.2007
(73) Patentinhaber: S & C Polymer Silicon- und Composite-Spezialitäten GmbH, 25335 Elmshorn (DE)
(72) Erfinder: Engelbrecht, Jürgen, S&C Polymer, Silicon- und, 25335 Elmshorn (DE); Görlich, K. J., S & C Polymer, Silicon- und, 25335 Elmshorn (DE)
(74) Vertreter: Forstmeyer, Dietmar
(86) Internationale Anmeldenummer: PCT/EP2005/013587
(87) Internationale Veröffentlichungsnummer: WO 2006/066822

(56) Entgegenhaltungen:
- WO-A-01/67878
- DE-A1- 2 043 279
- US-A- 3 201 407
- US-A1- 2004 005 277
- US-A1- 2004 067 204
- US-B1- 6 649 147

## Beschreibung

Die vorliegende Erfindung bezieht sich auf lichtaktivierbare "Zahnbleichmittel"-Zusammensetzungen sowie deren Verwendung zum Aufhellen von Zähnen. Die dentale Bleichmittelzusammensetzung der vorliegenden Erfindung umfasst Riboflavin und/oder Riboflavinderivate, die durch Anwendung von Strahlungsenergie für eine schnellere Zersetzung des im Bleichmittel enthaltenen Oxidationsmittels sorgen und damit die Bleichwirkung wirksam beschleunigen können. Ein oder mehrere Bestandteile der vorliegenden Erfindung können als Gele oder in Pastenform vorliegen und gegebenenfalls zusätzlich chemisch aktivierbar sein.

Die Verfärbung von Zähnen kann auf dem natürlichen Alterungsprozeß, dem Genuß von bestimmten Lebensmitteln und Tabak, auf Krankheiten, Verletzungen, Medikamenten und auf Umgebungsbedingungen beruhen oder angeboren sein. Da weiße oder helle Zähne im Allgemeinen als aesthetischer empfunden werden als dunkle oder verfärbte Zähne, besteht seit längerer Zeit ein großes Interesse an der Entwicklung von Materialien und Methoden zur Aufhellung von Zähnen.

Einige Zahnputzmittel wie Zahnpasten, -gele und -pulver enthalten aktiven Sauerstoff oder Wasserstoffperoxid freisetzende Bleichmaterialien. Solche Bleichmittel enthalten Peroxide, Percarbonate und Perborate von Alkali- und Erdalkalimetallen oder Komplexverbindungen, die Wasserstoffperoxid enthalten.

Eines der im Dentalbereich meist verwendeten Bleichmaterialien ist Percarbamid, auch Harnstoffperoxohydrat oder Harnstoff-Wasserstoffperoxid genannt, das im zahnärztlichen Bereich schon seit Jahrzehnten auch als orales Antiseptikum verwendet wird. Harnstoff selbst wird in der Literatur als Keratinisierungsmittel für das Zahnfleisch beschrieben; das Zahnbleichen war ein beobachteter Nebeneffekt bei ausgedehnten Kontaktzeiten. Andere Bleichmittel, wie z. B. Peroxyessigsäure und Natriumperborat, sind auf dem medizinischen, zahnärztlichen und kosmetischen Gebiet ebenfalls wohlbekannt.

In früheren Patenten wird auch offenbart, daß Wasserstoffperoxid, Percarbamid und andere Peroxide als Bleichmittel in Formulierungen von Zahnweißgelen verwendet werden, wobei Carboxypolymethylene und andere Verdicker zur Zubereitung von Gelen eingesetzt werden. Diese Gele können wasserfrei sein oder auf Wasser basieren.

Die auf dem Markt befindlichen Bleichgele, weltweit entsprechend dem Englischen auch als "Bleaching Gele" bezeichnet, werden in drei Kategorien eingeteilt, nämlich "Power Bleaching-", "Assisted Bleaching-" und "Home Bleaching-Gele". Um Zeit und Geld zu sparen ist das "Power Bleaching" in der Zahnarztpraxis die bevorzugte Methode für das Bleichen von verfärbten Zähnen.

U.S. Pat. No. 5,648,064 (Gaffar et al., 1995) beschreibt eine zweikomponentige Zahnpasta, in der der eine Bestandteil aktiven Sauerstoff und der zweite Bestandteil einen Mangan-Koordinationskomplex (Mangan-gluconat) als Aktivator enthält. Die erwähnten Farbstoffe dienen offensichtlich nur kosmetischen Zwecken.

U.S. Pat. No. 5,098,303 (Fischer, 1992), U.S. Pat. No. 5,234,342 (Fischer, 1993), U.S. Pat. No. 5,376,006 (Fischer, 1994), U.S. Pat. No. 5,725,843 (Fischer, 1998), U.S. Pat. No. 5,746,598 (Fischer, 1998), U.S. Pat. No. 5,759,038 (Fischer, 1998), U.S. Pat. No. 5,770,105 (Fischer, 1998), U.S. Pat. No. 5,785,527 (Fischer et al., 1998), U.S. Pat. No. 5,858,332 (Fischer et al., 1999), WO Pat. No. 9,937,236 (Fischer et al., 1999), U.S. Pat. No. 5,985,249 (Fischer, 1999), U.S. Pat. No. 6,036,943 (Fischer, 2000), WO Pat. No. 0,028,953 (Fischer et al., 2000), U.S. Pat. No. 6,086,855 (Fischer, 2000) und U.S. Pat. No. 6,183,251 (Fischer, 2001) beschreiben Zahnbleichmethoden und Zahnbleich- oder Fluoridgele, die als wirksames Agens Wasserstoffperoxid, Percarbamid, Natriumperborat, Benzoylperoxid, Glycerolperoxid sowie als Zusatzstoffe Wasser, Glycerol, Propylenglycol, Polyethylenglycol, Erythritol, Sorbitol, Mannitol, Carboxypolymethylene, Verdicker wie Xanthan, Talha, Tragant, Johannisbrotkernmehl, Guar, Ghatti, Furcelleran, Carrageen, Alginsäuren, Agar, Alginate, Proteine, Desensibilisierungsstoffe, Fluoride wie Natriummonofluorophosphat, Natriumfluorid und Zinkfluorid, antimicrobielle Stoffe wie Chlorhexidin, Tetracyclin, Cetylpyridiniumchlorid, Benzalkoniumchlorid, Cetylpyridiniumbromid, Methylbenzoat und Propylbenzoat enthalten.

In der Regel werden die Bleichgele durch Erhitzung, durch Bestrahlung von Strahlenenergie absorbierenden Materialien wie 9,10-bis(Phenylethynyl)-anthracen, Perylen, Naphtho[2,3-a]pyren, trans-4,4'-Diphenylstilben, 9,10-Diphenylanthracen, 5,12-bis(Phenylethynyl)-naphthacen, Coronen, Fluoranthen, Carotinoide, Bixin, Lycopen, 7-Diethylamino-4-methylcoumarin, Henna und Alizarin oder durch Zusatz Peroxid-zersetzender Agentien aktiviert.

EP Pat. No. 0,516,872 (Cornell, 1992) und JP Pat. No. 4,257,512 (Cornell, 1992) beschreibt eine Methode zum Zahnbleichen und eine dentale Bleichmischung. Letztere enthält ein wässriges Wasserstoffperoxidgel oder -paste und einen wasserfreien Bestandteil, der aus pyrogener Kieselsäure, einem Verdicker wie Celluloseether, Methylvinylester, einem Redoxindikator wie Guinea Grün und Phenolphthalein, die das Reaktionsende anzeigen sollen, und einem lichtaktivierten Katalysator wie Mangansulfat und Eisensulfat besteht.

EP Pat. No. 0,242,585 (Friedman, 1987) beschreibt eine Peroxidlösung, die durch Strahlungsenergie im Bereich von 320 bis 420 nm und von 500 bis 1200 nm aktiviert wird.

EP Pat. No. 1,224,925 (Banerjee & Friedman, 2002) beschreibt die Zusammensetzung eines Bleichmittels, das Manganchlorid, Mangancitrat, Eisensulfat, Natriumcarbonat, Natriumhydrogencarbonat und Catalase als Aktivatoren enthalten kann. Als Farbstoffe, die im Bereich von 360 bis 500 nm Licht absorbieren, sind FD & C Red 40 oder FD & C Yellow 5 oder FD & C Yellow 6 angegeben.

U.S. Pat. No. 6,116,900 (Ostler, 2000) beschreibt eine Doppelkammer-Mischvorrichtung und eine Zusammensetzung, die Peroxide wie Percarbamid, Calciumhydrogencarbonatperoxid, Natriumhydrogencarbonatperoxid und Wasserstoffperoxid, und Salze zur Änderung des pH-Werts auf einen Bereich von pH 7 bis pH 11, wie Natriumhydroxid, Calciumcarbonat und Calciumhydrogencarbonat, enthält. Zusätzlich sind als Farbindikator Thymolblau und als Farbstoff zur Verschiebung der Absorption der Lichtenergie von Blau nach Blau-Grün (400-550 nm) β-Carotin vorhanden. Anstelle eines Farbstoffes kann auch ein inertes gefärbtes Material wie Glasperlen oder pyrogene Kieselsäure für die Umwandlung von Lichtenergie in Hitze verwendet werden.

U.S. Pat. No. 5,645,428 (Yarborough, 1997) beschreibt eine Bleichmethode mit Peroxiden und Katalysatoren unter Anwendung eines Argonlasers und eines Kohlendioxidlasers zur Beschleunigung des Peroxidabbaus.

U.S. Pat. No. 6,149,895 (Kutsch, 2000) benennt eine Bleichmittelpaste, bei der ein roter Farbstoff (als Beispiel sind Chinaldinrot und Säurerot 92 genannt) in einem Lösemittel gelöst und mit einem inerten, anorganischen Trägermaterial gemischt wird. Diese Paste wird mit sichtbarem Licht von 400-550 nm oder einem Argonlaser bestrahlt. Bei Auftreten des aktiven Sauerstoffs entfärbt sich die Bleichpaste, so dass die Behandlungsdauer genau eingestellt werden kann.

U.S. Pat. No.5,766,011 (Sibner, 1998) beschreibt ein Bleichmittel, das einen pH-Wert von circa 9,5 bis 10 aufweist und aus Wasserstoffperoxid, einer inerten Siliciumverbindung, Natriumhydroxid und einem pigmentiertem Material besteht. Diese Zusammensetzung führt durch Anwendung eines Argonlasers zu verkürzter Kontaktzeit an den Zähnen.

U.S. Pat. No. 6,030,222 (Tarver, 2000) beschreibt eine Farbstoffmischung zur Aufhellung von Zähnen. Dieses Bleichmittel enthält eine Mischung von violetten und blauen Farbstoffen zur Absorption von Licht vom sichtbaren Bereich zum violetten bis blau-violetten Bereich, ein Peroxid, ein hydrophiles Lösemittel wie Wasser, Glycerol, Alkohole, Polyole, Ketone, Aldehyde, Carboxylsäuren, Salze der Carboxylsäuren und Amine, ein Geliermittel wie Polycarboxylsäuren, Salze der Polycarboxylsäuren, Polysaccharide, Proteine, Polyalkylenoxide und pyrogene Kieselsäure.

Eine Aufgabe der vorliegenden Erfindung war demgegenüber die Bereitstellung einer neuen und verbesserten Bleichmittelzusammensetzung für Zähne, die bei geringer Anwendungszeit eine effektive Bleichwirkung ermöglicht, und sowohl als Einkomponenten- als auch als Zweikomponentensystem anwendbar ist.

Diese Aufgabe wird durch eine dentale Bleichmittelzusammensetzung gelöst, die ein Oxidationsmittel (a), insbesondere ein Peroxid, und Riboflavin und/oder Riboflavinderivat(e) (b) umfasst.

Bevorzugt liegt das Oxidationsmittel wie das Peroxid (a) als Gel oder Paste, insbesondere als Gel vor.

Dadurch kann dentale Bleichmittel leichter und homogener zubereitet, genauer dosiert und leichter auf die zu behandelnden Zähne aufgebracht werden.

Gegebenenfalls kann die Bleichmittelzusammensetzung außerdem einen Aktivator (c) enthalten, der die Bleichwirkung des Peroxids bzw. des Peroxidgels (a) aktivieren kann. Bevorzugt liegt der Aktivator als Gel oder Paste, insbesondere als Gel vor, wodurch die Zubereitung, Dosierung und Anwendung des Bleichmittels weiter erleichtert bzw. verbessert wird.

Weiter bevorzugt liegt die gesamte Bleichmittelzusammensetzung als Gel oder in Form einer Paste vor.

Gemäß einer bevorzugten Ausführungsform liegen die Bestandteile (a) und (b) als Mischung vor (Einkomponenten-Material).

Gemäß einer weiteren bevorzugten Ausführungsform liegen die Bestandteile (a) und (c) getrennt voneinander vor bzw. werden getrennt voneinander eingesetzt, während der Bestandteil (b) in Mischung mit den Bestandteilen (a) und/oder (c) vorliegen kann (Zweikomponenten-Material).

Erfindungsgemäß wird das Oxidationsmittel (a), bezogen auf die gesamte Bleichmittelzusammensetzung, z.B. in einer Menge von 1-90, vorzugsweise in einer Menge von 2-70 und besonders bevorzugt von 5-55 Gew.-% (alle %-Angaben der vorliegenden Erfindung beziehen sich auf das Gewicht, sind also Gewichtsprozente) eingesetzt.

Weiter bevorzugt wird der Bestandteil (b), bezogen auf die gesamte Bleichmittelzusammensetzung, in einer Menge von 0,005-15 Gew.-%, bevorzugt in einer Menge von 0,01-10 Gew.-% und stärker bevorzugt von 0,05-5 Gew.-% eingesetzt.

Ferner kann der Bestandteil (c), bezogen auf die gesamte Bleichmittelzusammensetzung, z.B. in einer Menge von 0,01-15 Gew.-%, bevorzugt von 0,02-10 Gew.-%, und besonders bevorzugt von 0,05-5 Gew.-% eingesetzt werden.

Bevorzugt werden die beiden Bestandteile (a) und (c) direkt vor der Verwendung zum Bleichen von Zähnen gemischt.

Weiter bevorzugt ist eine Doppelkammermischvorrichtung, wobei eine Kammer das Peroxidgel (a), und die andere Kammer das Aktivatorgel (c) enthält, wobei Komponente (b) in einer oder beiden Kammern enthalten ist.

Es stellte sich heraus, dass Riboflavin sowie Riboflavinderivate eine überraschend hohe Effektivität bei der Zersetzung des im Bleichmittel enthaltenen Oxidationsmittels durch Lichtenergie aufweisen und somit die Bleichwirkung wirksam beschleunigen können.

Das dentale Bleichmittel dieser Erfindung umfasst ein Oxidationsmittel (a), insbesondere ein, zwei oder mehrere oral kompatible Oxidationsmittel, wie z.B. ein Peroxid. Beispiele für Peroxide und/oder Bestandteile von Peroxidmischungen, die bei der Zubereitung des Bleichmittels verwendet werden können, umfassen Wasserstoffperoxid, Percarbamid, Natriumperborat, Kaliumperoxomonosulfat, Kaliumchlorat, Kaliumpercarbonat, Natriumpercarbonat, Calciumperoxid, Magnesiumperoxid, Perphosphate, Persilicate, Benzoylperoxid, Glycerolperoxid, Calciumhydrogencarbonatperoxid und Natriumhydrogencarbonatperoxid. Wasserstoffperoxid, Percarbamid, Natriumperborat und Kaliumperoxomonosulfat werden als Bleichmittel bevorzugt. Wasserstoffperoxid, Percarbamid, Natriumperborat und/oder Kaliumperoxomonosulfat und/oder Mischungen von einer, zwei oder mehreren dieser Verbindungen sind vorzugsweise in der Bleichmittelzusammensetzung vorzugsweise in einer Menge von 5-70 Gewichtsprozent, insbesondere in einer Menge von 5-55 Gewichtsprozent vorhanden. Vorzugsweise weist das Peroxid bzw. Peroxidgel einen pH Wert im Bereich von 2-7 auf.

Der gegebenenfalls vorhandene weitere Bestandteil (c) ist ein Aktivator, insbesondere ein Aktivatorgel. Der Ausdruck Aktivatorgel bezeichnet Verbindungen und/oder Zusammensetzungen, welche die Bleichwirkung des Oxidationsmittels aktivieren können. Aktivatorgele können alkalische Gele sein. Sie enthalten bevorzugt Alkali- und/oder Erdalkalimetallsalze. Als Aktivatoren oder Zersetzungskatalysatoren können aber auch Salze oder Komplexe insbesondere aus der Gruppe Kupfer, Mangan und/oder Eisen ausgewählt werden, besonders bevorzugt Organometall-Komplexe oder -Salze wie z.B. Acetylacetonate, Gluconate, Lactate, Fumarate, Naphthensäuresalze, Metallocene, Oxalate, Citrate, Sulfate, Oxide, Acetate und/oder Mischungen davon. Sie zeigen entsprechend ihrem chemischen Charakter heftige bis milde Reaktionen bei der Zersetzung der Oxidationsmittel, insbesondere der Peroxide. Bevorzugt werden bei alkalischen Gelen pH-Werte von 8 bis 11. Metallkomplexe bzw. Salze werden vorzugsweise in Mengen von 0,01-15 Gewichtsprozent, besonders bevorzugt in Mengen von 0,05-5 Gewichtsprozent, bezogen auf die gesamte Bleichmittelzusammensetzung, eingesetzt.

Vorzugsweise enthalten das Peroxidgel und/oder das Aktivatorgel ein Grundlagenmaterial und/oder einen Verdicker, wobei bevorzugt wird, wenn beide Bestandteile ein Grundlagenmaterial und/oder einen Verdicker enthalten.

Als Gelbildner oder Verdicker zur Herstellung des Peroxidgels wie auch des optionalen Aktivatorgels können beispielsweise verwendet werden: Cellulosepolymere, Polycarbonsäuren, pyrogenes Siliciumdioxid, Poly(meth)acrylsäuren, Polysaccharide, Polyvinylbutyrale, Alginate, Coumaronharze, Schellack, Xanthan, Tragant, Guar, Carrageen, Alginsäuren etc. und/oder Mischungen davon. Sie können in den Bestandteilen, bezogen auf die gesamte Bleichmittelzusammensetzung in einer Menge von 0,01-20 Gewichtsprozent, vorzugsweise in einer Menge von 0,05-15 Gewichtsprozent vorhanden sein.

Als Grundlagenmaterial zur Herstellung von stabilen Gelen wird oft Wasser oder Wasser in Kombination mit anderen Verbindungen oder Mischungen verwendet. Solche Verbindungen oder Mischungen umfassen aus Polyole wie Polyethylenglycol, Sorbitol, Polypropylenglycol, Propylenglycol, Glycerol, Ethanol, Aceton, Ether, Essigsäureester, Xylit und andere und/oder Mischungen davon. Polyole wie Glycerol und/oder Propylenglycol und/oder demineralisiertes Wasser werden in dieser Erfindung bevorzugt. Sie sind entweder allein oder als Mischungen in einer Menge, bezogen auf die gesamte Bleichmittelzusammensetzung, von 0,1-98 Gewichtsprozent, und bevorzugt in einer Menge von 0,5-95 Gewichtsprozent vorhanden.

Ferner enthält die Bleichmittelzusammensetzung der vorliegenden Erfindung Riboflavin und/oder Riboflavinderivat(e). Dieser Bestandteil (b) kann dem Oxidationsmittel (a) und/oder dem Aktivator (c) zugesetzt werden. Durch Bestandteil (b) wird Lichtenergie in Wärme umgewandelt und somit die Bleichwirkung der dentalen Bleichmittelzusammensetzung verbessert und beschleunigt. Für die Wirkung ist es nicht wesentlich, in welchem Bestandteil das Riboflavin und/oder die Riboflavinderivate vorhanden sind. Es wird jedoch bevorzugt der Bestandteil gewählt, in dem es am stabilsten vorliegt. Die Menge an Riboflavin und/oder Riboflavinderivaten beträgt 0,001-15 Gewichtsprozent, vorzugsweise 0,005-10 Gewichtsprozent.

Beispiele für Riboflavinderivate sind: Riboflavin-5'-phosphat, Riboflavin-5'-malonat; bevorzugte Riboflavinderivate sind Riboflavin-5'-phosphat und Riboflavin-5'-malonat.

Die Zusammensetzungen der vorliegenden Erfindung können weitere Bestandteile enthalten, wie z. B. Stabilisatoren wie Alkalimetallpolyphosphate, Alkalimetallpyrophosphate, Ethylendiamintetraessigsäure und ihre Salze, Weinsäure und ihre Salze, Zitronensäure und ihre Salze, Gluconsäure und ihre Salze, Triethanolamin, Zinnnitrat, Adipinsäure, Zinnphosphat, Bernsteinsäure etc., wie z. B. Bestandteile, die den pH-Wert verändern wie Alkali- und Erdalkalimetallsalze, wie z.B. Vitamine als Entzündungshemmer, sowie Aromastoffe wie z.B. Pfefferminz, Vanillin etc., Farbstoffe zur Einfärbung und als Indikatoren, Konservierungsstoffe, Fluoridderivate, Netzmittel etc. Sie können sowohl allein als auch in Mischungen in den Gelen dieser Erfindung vorhanden sein.

### Beispiele

Um die überraschenderweise hohe Effektivität von Riboflavin und/oder Riboflavinderivaten zu demonstrieren, wurde der Temperaturanstieg während einer Bestrahlung gemessen. Als zahnärztliches Lichthärtegerät wurde das PowerPac Lichtgerät von American Dental Technologies verwendet. Die gemessenen Temperaturanstiege während der Bestrahlung sind in Tabelle 1 aufgelistet.

### Einkomponenten-Gele

Als Grundlagenmaterial wurde Glycerol und als Verdicker pyrogenes Siliciumdioxid verwendet.

| | | |
|---|---|---|
| **Vergleichsbeispiel 1:** | Glycerol | 73,00 % w/w |
| | Verdicker | 5,00 % w/w |
| | Percarbamid | 22,00 % w/w |
| **Beispiel 2:** | Glycerol | 73,00 % w/w |
| | Verdicker | 4,99 % w/w |
| | Percarbamid | 22,00 % w/w |
| | Riboflavin-5'-phosphat | 0,01 % w/w |
| **Beispiel 3:** | Glycerol | 83,00 % w/w |
| | Verdicker | 5,99 % w/w |
| | Percarbamid | 11,00 % w/w |
| | Riboflavin-5'-phosphat | 0,01 % w/w |

### Zweikomponenten-Gele

Als Grundlagenmaterial wurde Glycerol und als Verdicker pyrogenes Siliciumdioxid verwendet.

| | | | |
|---|---|---|---|
| **Vergleichsbeispiel 4:** | **a)** | Glycerol | 83,00 % w/w |
| | | Verdicker | 6,00 % w/w |
| | | Percarbamid | 11,00 % w/w |
| | **b)** | Glycerol | 93,00 % w/w |
| | | Verdicker | 6,40 % w/w |
| | | Fe(II)-sulfat | 0,60 % w/w |
| **Beispiel 5:** | **a)** | Glycerol | 83,00 % w/w |
| | | Verdicker | 5,99 % w/w |
| | | Percarbamid | 11,00 % w/w |
| | | Riboflavin-5'-phosphat | 0,01 % w/w |
| | **b)** | Glycerol | 93,00 % w/w |
| | | Verdicker | 6,40 % w/w |
| | | Fe(II)-sulfat | 0,60 % w/w |
| **Beispiel 6:** | **a)** | Glycerol | 73,00 % w/w |
| | | Verdicker | 4,99 % w/w |
| | | Percarbamid | 22,00 % w/w |
| | | Riboflavin-5'-phosphat | 0,01 % w/w |
| | **b)** | Glycerol | 93,00 % w/w |
| | | Verdicker | 6,40 % w/w |
| | | Fe(II)-sulfat | 0,60 % w/w |

### Ergebnisse:

**Tabelle 1: Temperaturanstieg von Ein- und Zweikomponenten-Bleichmaterial bei Bestrahlung mit dem PowerPac Gerät**

| Bleichmaterialien | PowerPac ΔT |
|---|---|
| Einkomponentengele | |
| Vergleichsbeispiel 1 | ca. 6,9°C |
| Beispiel 2 | ca. 10,0°C |
| Beispiel 3 | ca. 11,7°C |

| Zweikomponentengele | |
|---|---|
| Vergleichsbeispiel 4a) + 4b) | ca. 6,7°C |
| Beispiel 5a) + 5b) | ca. 8,5°C |
| Beispiel 6a) + 6b) | ca. 8,8°C |
| QuasarBrite^{™} | ca. 4,2°C |
| (Vergleichsbeispiel 7) | |

Die Bestrahlungszeiten für die (Vergleichs-)Beispiele 1 bis 6 und QuasarBrite^{™} (einem lichtaktiviertem "Bleach"-Produkt als Vergleich, welches zur Licht-Aktivierung Thermal Absorption Crystals^{™} enthält) betrugen 30 Sekunden mit dem PowerPac Gerät. Die (Vergleichs-)Beispiele 4 bis 6 wurden kurz nach der chemischen Reaktion des Zweikomponenten-Materials bestrahlt. In den zuletzt genannten Beispielen wurden handelsübliche 1:1 Doppelkammerspritzen (Fa. MixPac, Schweiz) verwendet, so daß der eigentliche Peroxidgehalt nur die Hälfte von den Mengen in den Beispielen beträgt. Tabelle 1 zeigt damit die überlegene Effektivität des eingesetzten Riboflavinderivates.

## Patentansprüche

1. Dentale Bleichmittelzusammensetzung, **dadurch gekennzeichnet, dass** sie (a) ein Oxidationsmittel und (b) Riboflavin und/oder ein Riboflavinderivat umfasst.

2. Bleichmittelzusammensetzung nach Anspruch 1, **dadurch gekennzeichnet, dass** das Oxidationsmittel (a) ein Peroxid ist.

3. Bleichmittelzusammensetzung nach Anspruch 1 oder 2, **dadurch gekennzeichnet, dass** sie zusätzlich (c) einen Aktivator umfasst.

4. Bleichmittelzusammensetzung nach einem der Ansprüche 1 bis 3, **dadurch gekennzeichnet, dass** zumindest einer der Bestandteile (a) und (c) als Paste oder Gel vorliegt.

5. Bleichmittelzusammensetzung nach einem der vorstehenden Ansprüche, **dadurch gekennzeichnet, dass** sie als Gel oder Paste vorliegt.

6. Bleichmittelzusammensetzung nach Anspruch 4 oder 5, **dadurch gekennzeichnet, dass** Bestandteil (a) einen pH von 2 bis 7 aufweist.

7. Bleichmittelzusammensetzung nach einem der Ansprüche 2 bis 6, **dadurch gekennzeichnet, daß** das Peroxid (a) Wasserstoffperoxid, Percarbamid, Natriumperborat oder Kaliumperoxomonosulfat oder eine Mischung davon ist.

8. Bleichmittelzusammensetzung nach einem der Ansprüche 2 bis 7, **dadurch gekennzeichnet, dass** Bestandteil (a) in einer Menge von 2-90 Gewichtsprozent, vorzugsweise in einer Menge von 5-55 Gewichtsprozent bezogen auf die gesamte Bleichmittelzusammensetzung, enthalten ist.

9. Bleichmittelzusammensetzung nach einem der Ansprüche 3 bis 8, **dadurch gekennzeichnet, daß** der Aktivator (c) zumindest einen alkalischen Zusatz enthält.

10. Bleichmittelzusammensetzung nach Anspruch 9, **dadurch gekennzeichnet, daß** der alkalische Zusatz ein Alkali- oder ein Erdalkalimetallsalz enthält oder daraus besteht.

11. Bleichmittelzusammensetzung nach einem der Ansprüche 3 bis 8, **dadurch gekennzeichnet, daß** der Aktivator (c) zumindest ein Salz oder zumindest einen Komplex eines Metalls aus der Gruppe Kupfer, Mangan und Eisen enthält.

12. Bleichmittelzusammensetzung nach Anspruch 11**, dadurch gekennzeichnet, daß** der Aktivator (c) ein Acetylacetonat, Gluconat, Lactat, Fumarat, Naphthensäuresalz, Metallocen, Oxalat, Citrat, Sulfat, Oxid, Acetat oder eine Mischung davon ist.

13. Bleichmittelzusammensetzung nach Anspruch 11 oder 12, **dadurch gekennzeichnet, daß** der Aktivator (c) in einer Menge von 0,01-15 Gewichtsprozent, bezogen auf die gesamte Bleichmittelzusammensetzung, enthalten ist.

14. Bleichmittelzusammensetzung nach Anspruch 11 oder 12, **dadurch gekennzeichnet, daß** der Aktivator (c) in einer Menge von 0,05-5 Gewichtsprozent, bezogen auf die gesamte Bleichmittelzusammensetzung, enthalten ist.

15. Bleichmittelzusammensetzung nach einem der Ansprüche 1 bis 14, **dadurch gekennzeichnet, daß** das Riboflavinderivat (b) Riboflavin-5'-phosphat oder Riboflavin-5'-malonat ist.

16. Bleichmittelzusammensetzung nach einem der Ansprüche 1 bis 15, **dadurch gekennzeichnet, dass** Bestandteil (b) in einer Menge von 0,005-15 Gewichtsprozent, bezogen auf die gesamte Bleichmittelzusammensetzung, enthalten ist.

17. Bleichmittelzusammensetzung nach einem der Ansprüche 4 bis 16, **dadurch gekennzeichnet, dass** Bestandteil (b) im Peroxidgel (a) oder im Aktivatorgel (c) oder in beiden vorliegt.

18. Ein Satz von Teilen (kit of parts) nach einem der Ansprüche 3 bis 17, **dadurch gekennzeichnet, dass** Bestandteil (a) getrennt von der Bestandteil (c) vorliegt.

19. Doppelkammermischvorrichtung mit statischem Mischer, das eine Bleichmittelzusammensetzung nach einem der Ansprüche 3 bis 17 enthält, **dadurch gekennzeichnet, dass** sie in einer Kammer Bestandteil (a), in der anderen Kammer Bestandteil (c) und in zumindest einer der beiden Kammern Bestandteil (b) enthält.

20. Verwendung von (a) einem Oxidationsmittel und (b) Riboflavin und/oder Riboflavinderivaten zur Herstellung von Zusammensetzungen zum Aufhellen von Zähnen.

21. Verwendung von (a) einem Oxidationsmittel und (b) Riboflavin und/oder Riboflavinderivaten zum Aufhellen von Zähnen.

## Claims

1. Dental bleaching agent composition, **characterised in that** it comprises (a) an oxidising agent and (b) riboflavin and/or a riboflavin derivative.

2. Bleaching agent composition according to claim 1, **characterised in that** the oxidising agent (a) is a peroxide.

3. Bleaching agent composition according to claim 1 or 2, **characterised in that** it additionally comprises (c) an activator.

4. Bleaching agent composition according to any one of claims 1 to 3, **characterised in that** at least one of the constituents (a) and (c) is in the form of a paste or gel.

5. Bleaching agent composition according to any one of the preceding claims, **characterised in that** it is in the form of a gel or paste.

6. Bleaching agent composition according to claim 4 or 5, **characterised in that** constituent (a) has a pH of from 2 to 7.

7. Bleaching agent composition according to any one of claims 2 to 6, **characterised in that** the peroxide (a) is hydrogen peroxide, percarbamide, sodium perborate or potassium peroxomonosulfate or a mixture thereof.

8. Bleaching agent composition according to any one of claims 2 to 7, **characterised in that** constituent (a) is contained in an amount of from 2 to 90 % by weight, preferably in an amount of from 5 to 55 % by weight, based on the total bleaching agent composition.

9. Bleaching agent composition according to any one of claims 3 to 8, **characterised in that** the activator (c) comprises at least one alkaline additive.

10. Bleaching agent composition according to claim 9, **characterised in that** the alkaline additive comprises or consists of an alkali metal salt or an alkaline earth metal salt.

11. Bleaching agent composition according to any one of claims 3 to 8, **characterised in that** the activator (c) comprises at least one salt or at least one complex of a metal from the group copper, manganese and iron.

12. Bleaching agent composition according to claim 11, **characterised in that** the activator (c) is an acetylacetonate, gluconate, lactate, fumarate, naphthenic acid salt, metallocene, oxalate, citrate, sulfate, oxide, acetate or a mixture thereof.

13. Bleaching agent composition according to claim 11 or 12, **characterised in that** the activator (c) is contained in an amount of from 0.01 to 15 % by weight, based on the total bleaching agent composition.

14. Bleaching agent composition according to claim 11 or 12, **characterised in that** the activator (c) is contained in an amount of from 0.05 to 5 % by weight, based on the total bleaching agent composition.

15. Bleaching agent composition according to any one of claims 1 to 14, **characterised in that** the riboflavin derivative (b) is riboflavin-5'-phosphate or riboflavin-5'-malonate.

16. Bleaching agent composition according to any one of claims 1 to 15, **characterised in that** constituent (b) is contained in an amount of from 0.005 to 15 % by weight, based on the total bleaching agent composition.

17. Bleaching agent composition according to any one of claims 4 to 16, **characterised in that** constituent (b) is present in the peroxide gel (a) or in the activator gel (c) or in both.

18. A kit of parts according to any one of claims 3 to 17, **characterised in that** constituent (a) is present separately from constituent (c).

19. Double-chamber mixing device having a static mixer, comprising a bleaching agent composition according to any one of claims 3 to 17, **characterised in that** it comprises in one chamber constituent (a), in the other chamber constituent (c) and in at least one of the two chambers constituent (b).

20. Use of (a) an oxidising agent and (b) riboflavin and/or riboflavin derivatives in the preparation of compositions for whitening teeth.

21. Use of (a) an oxidising agent and (b) riboflavin and/or riboflavin derivatives in whitening teeth.

## Revendications

1. Composition de blanchiment des dents, **caractérisée en ce qu'**elle comprend (a) un agent oxydant et (b) de la riboflavine et/ou un dérivé de riboflavine.

2. Composition de blanchiment selon la revendication 1, **caractérisée en ce que** l'agent oxydant (a) est un peroxyde.

3. Composition de blanchiment selon la revendication 1 ou 2, **caractérisée en ce qu'**elle contient en outre (c) un activateur.

4. Composition de blanchiment selon l'une quelconque des revendications 1 à 3, **caractérisée en ce qu'**au moins l'un des composants (a) et (c) se présente sous forme de pâte ou de gel.

5. Composition de blanchiment selon l'une quelconque des revendications précédentes, **caractérisée en ce qu'**elle se présente sous forme de gel ou de pâte.

6. Composition de blanchiment selon la revendication 4 ou 5, **caractérisée en ce que** le composant (a) présente un pH de 2 à 7.

7. Composition de blanchiment selon l'une quelconque des revendications 2 à 6, **caractérisée en ce que** le peroxyde (a) est le peroxyde d'hydrogène, le percarbamide, le perborate de sodium ou le peroxomonosulfate de potassium ou un mélange de ceux-ci.

8. Composition de blanchiment selon l'une quelconque des revendications 2 à 7, **caractérisée en ce que** le composant (a) est contenu en une quantité de 2 à 90 % en poids, de préférence en une quantité de 5 à 55 % en poids par rapport à la composition de blanchiment totale.

9. Composition de blanchiment selon l'une quelconque des revendications 3 à 8, **caractérisée en ce que** l'activateur (c) contient au moins un additif alcalin.

10. Composition de blanchiment selon la revendication 9, **caractérisée en ce que** l'additif alcalin contient ou est constitué d'un sel de métal alcalin ou d'un sel de métal alcalinoterreux.

11. Composition de blanchiment selon l'une quelconque des revendications 3 à 8, **caractérisée en ce que** l'activateur (c) contient au moins un sel ou au moins un complexe d'un métal du groupe comprenant le cuivre, le manganèse et le fer.

12. Composition de blanchiment selon la revendication 11, **caractérisée en ce que** l'activateur (c) est un acétyl-acétonate, un gluconate, un lactate, un fumarate, un sel d'acide naphténique, un métallocène, un oxalate, un citrate, un sulfate, un oxyde, un acétate ou un mélange de ceux-ci.

13. Composition de blanchiment selon la revendication 11 ou 12, **caractérisée en ce que** l'activateur (c) est contenu en une quantité de 0,01 à 15 % en poids par rapport à la composition de blanchiment totale.

14. Composition de blanchiment selon la revendication 11 ou 12, **caractérisée en ce que** l'activateur (c) est contenu en une quantité de 0,05 à 5 % en poids par rapport à la composition de blanchiment totale.

15. Composition de blanchiment selon l'une quelconque des revendications 1 à 14, **caractérisée en ce que** le dérivé de riboflavine (b) est le riboflavine-5'-phosphate ou le riboflavine-5'-malonate.

16. Composition de blanchiment selon l'une quelconque des revendications 1 à 15, **caractérisée en ce que** le composant (b) est contenu en une quantité de 0,005 à 15 % en poids par rapport à la composition de blanchiment totale.

17. Composition de blanchiment selon l'une quelconque des revendications 4 à 16, **caractérisée en ce que** le composant (b) est contenu dans le gel de peroxyde (a) ou dans le gel activateur (c) ou dans les deux.

18. Trousse de pièces (kits of parts) selon l'une quelconque des revendications 3 à 17, **caractérisée en ce que** le composant (a) se présente séparé du composant (c).

19. Dispositif de mélange à deux chambres comportant un mélangeur statique qui contient une composition de blanchiment selon l'une quelconque des revendications 3 à 17, **caractérisé en ce qu'**il contient dans une chambre, le composant (a), dans l'autre chambre, le composant (c) et dans au moins l'une des deux chambres, le composant (b).

20. Utilisation de (a) un agent oxydant et (b) de la riboflavine et/ou des dérivés de riboflavine pour la production de compositions pour éclaircir les dents.

21. Utilisation de (a) un agent oxydant et (b) de la ribloflavine et/ou des dérivés de riboflavine pour éclaircir les dents.
